# EUROPEAN PATENT APPLICATION

(11) **EP 2 352 029 A1**
(43) Date of publication of application: **03.08.2011**
(21) Application number: 09826130.8
(22) Date of filing: 12.11.2009
(51) Int. Cl.: G01N 33/545, G01N 33/53, G01N 33/543

(54) **INSOLUBLE CARRIER FOR USE IN ANTI-PHOSPHOLIPID ANTIBODY MEASUREMENT REAGENT, ANTI-PHOSPHOLIPID ANTIBODY MEASUREMENT REAGENT, AND METHOD FOR MEASURING ANTI-PHOSPHOLIPID ANTIBODY**

(30) Priority: 12.11.2008 JP 2008289581
(71) Applicant: Sekisui Medical Co., Ltd., Tokyo 103-0027 (JP)
(72) Inventor: AKAMINE Takayuki, Ryugasaki-shi Ibaraki 301-0852 (JP); KITAHARA Shinichiro, Ryugasaki-shi Ibaraki 301-0852 (JP); OTA Tetsuya, Ryugasaki-shi Ibaraki 301-0852 (JP); ABE Takayuki, Ryugasaki-shi Ibaraki 301-0852 (JP)
(74) Representative: Hart-Davis, Jason
(86) International application number: PCT/JP2009/069273
(87) International publication number: WO 2010/055883

(57) **Abstract**

The present invention has an object to provide an insoluble carrier for an antiphospholipid antibody detection reagent having a high reactivity. The present invention also has an object to provide an antiphospholipid antibody detection reagent, and a method of detecting an antiphospholipid antibody. The present invention directs to an insoluble carrier for an antiphospholipid antibody detection reagent, having a zeta potential of lower than -45 mV in the case that the insoluble carrier is suspended in a 20 mmol/L aqueous sodium phosphate solution with a pH of 7.4 so that the resulting suspension has a solids concentration of 0.1%.

## Description

### TECHNICAL FIELD

The present invention relates to an insoluble carrier for an antiphospholipid antibody detection reagent having a high reactivity. The present invention also relates to an antiphospholipid antibody detection reagent and a method of detecting an antiphospholipid antibody.

### BACKGROUND ART

Immunoassays are used as methods of detecting a trace substance contained in blood, urine, and the like. The immunoassays can specifically detect at a high sensitivity the objective substance in a sample containing various substances, by making use of the specific, strong binding of an antigen and an antibody.

Still, in recent years, a higher sensitivity in immunoassays is strongly desired because there are increasing needs for measuring an ultra-trace substance such as a cancer marker, an antigen including viruses, and an antibody against bacteria and viruses contained in blood.

Examples of a method of improving the sensitivity of an immunoassay include the methods disclosed by the following Patent Documents. For example, Patent Document 1 discloses a method of measuring the zeta potential of an insoluble carrier for an immunoassay under a certain condition so as to select a carrier having a zeta potential of -20 mV or higher and lower than 0 mv, and then making the carrier physically adsorb a large amount of an antigen or antibody in an efficient manner. Patent Document 2 discloses a method of using a carrier in which the amount of sulfonic acid group per unit surface area of latex particles for an immunoassay is controlled to be in the range of 0.005 to 0.7 µmol/m².

Patent Document 1: Japanese Kokai Publication H07-270423 (JP-A H07-270423)
Patent Document 2: WO 03/005031

### DISCLOSURE OF INVENTION

### PROBLEMS TO BE SOLVED BY THE INVENTION

The present invention has an object to provide an insoluble carrier for an antiphospholipid antibody detection reagent having a high reactivity. Further, the present invention has an object to provide an antiphospholipid antibody detection reagent and a method of detecting an antiphospholipid antibody.

### MEANS FOR SOLVING THE PROBLEMS

The present inventors have found a problem in the method with use of a phospholipid adsorbed to an insoluble carrier that has a zeta potential of -20 mV or higher and lower than 0 mV. The problem is that in the above method, the antiphospholipid antibody cannot be detected at a high sensitivity, differently from the case that an ordinary antigen or antibody derived from a protein is adsorbed to an insoluble carrier. The present inventors have then made various studies on insoluble carriers, and compared the of making an insoluble carrier adsorb a phospholipid having a few hydrophilic portions, for insoluble carriers having the same particle size and different zeta potentials. As a result, the present inventors have found that an insoluble carrier having a lower zeta potential enables to produce an antiphospholipid antibody detection reagent having a higher reactivity, i.e., having a higher sensitivity, and thereby completed the present invention.
More specifically, one aspect of the present invention is an insoluble carrier for an antiphospholipid antibody detection reagent, having a zeta potential of lower than -45 mV in the case that the insoluble carrier is suspended in a 20 mmol/L aqueous sodium phosphate solution with a pH of 7.4 so that the resulting suspension has a solids concentration of 0.1%.
Other aspects of the present invention are an antiphospholipid antibody detection reagent, and a method of detecting an antiphospholipid antibody with use of the above insoluble carrier.

There is no clear theory that explains why an antiphospholipid antibody detection reagent having a high reactivity is produced by using an insoluble carrier having a low zeta potential. Insoluble carriers having a zeta potential closer to zero are said to have a high reactivity because an insoluble carrier having a zeta potential closer to zero is less repulsive and therefore tends to easily agglutinate. However, autoagglutination will take place when the potential is completely lost, that is, when the potential is zero. Hence, a certain level of potential is necessary. Further, the antigen-antibody reaction does not occur easily in the case that an antigen (or antibody) to be adsorbed to an insoluble carrier is at a position very close to the reacting antibody (or antigen) . Accordingly, a certain distance between an antigen and an antibody is considered to be necessary for a stable reaction.

In the case of making an insoluble carrier adsorb an antigen or antibody derived from a protein, the potentials derived from hydrophilic amino acid residues in the protein increase the potential on the insoluble carrier surface. Accordingly, an insoluble carrier should have a certain amount of potential after adsorbing an antigen or antibody derived from a protein even if the carrier originally has a zeta potential close to zero. In contrast, in the case of making an insoluble carrier adsorb a phospholipid, the potential of the carrier will not change much because phospholipids have only a few hydrophilic portions and thus have a potential close to zero. Accordingly, use of an insoluble carrier having a zeta potential close to zero in this case is not considered to provide a stable reaction. Also, with a larger absolute value of the zeta potential, the phospholipid molecules to be loaded onto the carrier may possibly be oriented in the direction suitable for the reaction with an antiphospholipid antibody.
The present invention is described in detail below.

An insoluble carrier for an antiphospholipid antibody detection reagent according to the present invention is an insoluble carrier having a zeta potential of lower than -45 mV in the case that the insoluble carrier is suspended in a 20 mmol/L aqueous sodium phosphate solution with a pH of 7.4 so that the resulting suspension has a solids concentration of 0.1%. If the insoluble carrier for an antiphospholipid antibody detection reagent according to the present invention has a zeta potential of lower than -45 mV, an antiphospholipid antibody detection reagent produced using the carrier will have a high reactivity. Here, the minimum value for the zeta potential is not particularly limited, but is practically about -100 mV. A preferable value for the zeta potential is -74 mV or lower.

The insoluble carrier for an antiphospholipid antibody detection reagent according to the present invention is not particularly limited, and examples thereof include organic polymer powders, microorganisms, blood cells, and cell membranes. Among those, organic polymer powders are preferable.
Examples of organic polymer powders include natural polymer powders and synthetic polymer powders.

The natural polymer powders are not particularly limited, and examples thereof include insoluble agarose, cellulose, and insoluble dextran.
The synthetic polymer powders are not particularly limited either, and examples thereof include polystyrene, styrene-sulfonic acid copolymers (styrene-sulfonate copolymers), styrene-(meth)acrylic acid copolymers, acrylonitrile-butadiene-styrene copolymers, vinyl chloride-(meth)acrylic ester copolymers, and vinyl acetate-(meth)acrylic ester copolymers.

The insoluble carrier for an antiphospholipid antibody detection reagent according to the present invention may be an insoluble carrier having a group such as sulfonic acid group or carboxyl group introduced on the surface thereof.
Among various insoluble carries, latex particles produced by uniformly dispersing microscopic particles of a synthetic polymer in an aqueous medium are preferable.

The latex particles are produced from a copolymer of a polymerizable monomer having phenyl group, and a polymerizable monomer having phenyl group and sulfonic acid group. The polymerizable monomer having phenyl group is not particularly limited, and examples thereof include styrene, divinylbenzene, ethylstyrene, α-methylstyrene, p-methylstyrene, p-ohlorostyrene, and chloromethyl styrene. Those polymerizable monomers having phenyl group may be used alone, or two or more of those polymerizable monomers may be uses in combination. Among these, styrene is preferable.

The polymerizable monomer having phenyl group and sulfonic acid group is not particularly limited as long as the monomer enables carrier particles to have sulfonic acid group on the surfaces thereof after polymerization. Examples of the monomer include styrene sulfonate, divinylbenzene sulfonate, ethyl styrene sulfonate, and α-methyl sulfonate. Further, these salts here are not particularly limited, and examples thereof include sodium salts, potassium salts, lithium salts, and ammonium salts. The polymerizable monomers having phenyl group and sulfonic acid group may be used alone, or two or more of the monomers may be used in combination. Among the monomers, styrene sulfonate is preferable, and sodium styrene sulfonate is more preferable.

The above latex particles can be produced by copolymerizing the above polymerizable monomer having phenyl group and the above polymerizable monomer having phenyl group and sulfonic acid group.
The polymerizable monomer having phenyl group and the polymerizable monomer having phenyl group and sulfonic acid group can be copolymerized by a conventionally known method. Examples of the conventionally known method include a method of putting the polymerizable monomer having phenyl group, the polymerizable monomer having phenyl group and sulfonic acid group, a polymerization initiator and, according to need, an emulsifier into a reaction vessel that has water charged therein as a solvent; and then stirring the mixture under nitrogen atmosphere.

The polymerization temperature in copolymerization of the polymerizable having phenyl group and the polymerizable polymer having phenyl group and sulfonic acid group is not particularly limited. The preferable lower limit of the polymerization temperature is 50°C and the preferable upper limit of the polymerization temperature is 100°C. A polymerization temperature of less than 50°C may not sufficiently progress the polymerization reaction. In contrast, a polymerization temperature exceeding 100°C may excessively increase the rate of polymerization, and thus may make it difficult to control the particle size. The more preferable lower limit of the polymerization temperature is 60°C and the more preferable upper limit of the polymerization temperature is 85°C. The common polymerization time is 5 to 50 hours, which differs depending on the conditions such as the compositions and concentrations of the polymerizable monomers, and the kind of the polymerization initiator.

The blending amount of the polymerizable monomer having phenyl group and sulfonic acid group to the polymerizable monomer having phenyl group needs to be set in consideration of the amount of sulfonic acid group on the surfaces of particles produced by copolymerization, and the particle sizes. The insoluble carrier for an antiphospholipid antibody detection reagent according to the present invention is to adsorb a low-charged phospholipid, not an ordinary antigen or antibody derived from a protein. Therefore, if the amount of sulfonic acid group is small, the charge repulsion between the particles after adsorption of a phospholipid by the carrier may be weak and thus spontaneous agglutination may occur; in this case, a stable reagent will not be produced. Accordingly, the amount of sulfonic acid group per unit surface area of latex particles is preferably 0.1 µmol/m² or larger. The blending amount of the polymerizable monomer having phenyl group to the polymerizable monomer having phenyl group and sulfonic acid group is preferably 0.02 to 0.2% by weight so that the amount of sulfonic acid group per unit surface area of copolymerized particles will be 0.1 to 0.7 µmol/m².
The amount of sulfonic acid group per unit surface area of latex particles can be determined by conductometric titration (Journal of Colloid and Interface Sciences. 49(3)425, 1974). If the above value is divided by the total surface area of the particles calculated from the determined particle sizes, the amount of sulfonic acid group per unit area can be determined.

The polymerization initiator is not particularly limited, and examples thereof include persulfates.
The persulfates are not particularly limited, and examples thereof include potassium persulfate, sodium persulfate, and ammonium peroxodisulfate.
The blending amount of the polymerization initiator is not particularly limited, and is usually in the range of 0.01 to 1% by weight with respect to the amount of the polymerizable monomers.

It is preferable that an emulsifier be not used because an emulsifier, when contained in the latex particles, may cause inconvenience such as inhibition of detection accuracy. However, an emulsifier may be used according to need, for example in the case that an emulsifier is required for adjustment of the amount of sulfonic acid group per unit surface area of latex particles.
The blending amount of the emulsifier is not particularly limited. Still, the maximum blending amount thereof is preferably 1% by weight, is more preferably 0.5% by weight, and is still more preferably 0.02% by weight with respect to the polymerizable monomer having phenyl group, regarding that the emulsifier is to be removed in a tail-end process after polymerization. The minimum blending amount of the emulsifier is preferably 0.01% by weight.

In copolymerization of the polymerizable monomer having phenyl group and the polymerizable monomer having phenyl group and sulfonic acid group, a polymerizable unsaturated monomer may be further added. The polymerizable unsaturated monomer is not particularly limited as long as the monomer is usable in common radical polymerization, and examples thereof include (meth)acrylic acid, (meth)acrylic esters, styrene derivatives, (meth)acrylonitrile, (meth)acrylic acid amide, vinyl halides, vinyl esters, (meth)acrolein, maleic acid derivatives, and fumaric acid derivatives.
Here, "(meth)acrylic acid" refers to an acrylic acid or a methacrylic acid.

Further, in copolymerization of the polymerizable monomer having phenyl group and the polymerizable monomer having phenyl group and sulfonic acid group, various salts may be added to improve the polymerization stability, according to need. The salts are not particularly limited as long as being usable in common radical polymerization, and examples thereof include magnesium sulfate, calcium sulfate, disodium sulfate, dipotassium sulfate, sodium dihydrogenphosphate, disodium hydrogenphosphate, potassium dihydrogenphosphate, dipotassium hydrogenphosphate, sodium chloride, and potassium chloride.

The average particle size of the latex particles used in detection may be appropriately selected according to the detection method and the detection device used. Generally, the minimum average particle size is preferably 0.01 µm and the maximum average particle size is preferably 1.5 µm. An average particle size of the latex particles of less than 0.01 µm may cause only a small amount of optical change after agglutination and thus may not provide the sensitivity required for detection. Also, it may take a long time for centrifugation to be completed in reagent preparation, and thereby the reagent cost may be increased. In contrast, an average particle size of the latex particles exceeding 1.5 µm may cause the amount of optical change to be out of detestable range after agglutination of the latex particles in the case that a sample has a high concentration of the substance to be detected, whereby a determined amount of optical change may not reflect the amount of the substance to be detected. In terms of the sensitivity of the antiphospholipid antibody detection in the case of using a general-purpose automatic analyzer, the minimum average particle size of the latex particles used in the present invention is preferably 0.2 µm, the maximum average particle size is preferably 0.5 µm, the minimum average particle size is more preferably 0.3 µm, and the maximum average particle size is more preferably 0.4 µm. The average particle size can be determined by an image analysis using a transmission electron microscope.

The latex particles may have any coefficient of variation (hereinafter also referred to as a CV value (%)) of the particle size, and the preferable upper limit of the CV value (%) is 10%. The CV value (%) of the particle size of the latex particles exceeding 10% may lead to a poor lot reproducibility in reagent preparation, thereby decreasing the reproducibility of the detection reagent. The more preferable upper limit of the CV value (%) of the particle size of the latex particles is 5%, and the even more preferable upper limit of the CV value (%) of the particle size of the latex particles is 3%.
The CV value (%) of the particle size can be calculated by the following formula.
CV value (%) of particle size = standard deviation of particle sizes/average particle size × 100

The latex particles may be of only one kind of particles, or may be of two or more kinds of latex particles as long as the particles have a zeta potential of lower than -45 mV in the case that the particles are suspended in a 20 mmol/L aqueous sodium phosphate solution with a pH of 7.4 so that the resulting suspension has a solids concentration of 0.1%. Use of two or more kinds of latex particles enables to detect an antigen-antibody reaction in a sample having any concentration within a wide concentration range from a low concentration to a high concentration, at a high sensitivity and high accuracy. Further, such use particularly enables to produce a detection reagent that is suitable for measurement with optical measurement devices such as a spectrophotometer, a turbidimeter, and a light scattering measurement device.

Another aspect of the present invention is an antiphospholipid antibody detection reagent for antiphospholipid antibody detection, which comprises: an insoluble carrier supporting a phospholipid antigen; and a buffer solution, wherein the insoluble carrier before supporting a phospholipid antigen has a zeta potential of lower than -45 mV in the case that the insoluble carrier is suspended in a 20 mmol/L aqueous sodium phosphate solution with a pH of 7.4 so that the resulting suspension has a solids concentration of 0.1%.

The antiphospholipid antibody detection reagent of the present invention contains an insoluble carrier supporting a phospholipid antigen.
The phospholipid antigen is not particularly limited, and preferable examples thereof include a phospholipid antigen containing cardiolipin, phosphatidylcholine, and cholesterol.
The cardiolipin is preferably refined from bovine heart, but may be chemically synthesized.
The phosphatidylcholine is preferably refined from chicken egg yolk, but may be lecithin having a phosphatidylcholine content of 60 to 80%. Alternatively, the phosphatidylcholine may be extracted from bovine heart, a soybean, or the like, or may be chemically synthesized.
The cholesterol may be from animals, or may be chemically synthesized.

The blending ratio of the cardiolipin, phosphatidylcholine, and cholesterol is not particularly limited, and it is preferable that a phospholipid antigen contain 8 to 12 mg of phosphatidylcholine and 1 to 5 mg of cholesterol per 1 mg of cardiolipin.

The method of providing the phospholipid antigen to the insoluble carrier is not particularly limited, and examples thereof include a method of providing a phospholipid antigen by making use of physical and/or chemical binding by a conventionally known method.

The antiphospholipid antibody detection reagent of the present invention contains a buffer solution.
The buffer solution has a function of dispersing or suspending the latex particles having a phospholipid antigen.
The buffer solution is not particularly limited, and examples thereof include phosphate buffer solutions, glycine buffer solutions, Tris-salt buffer solutions, and Good' s buffer solutions.
The buffer solution may have any pH value, and the preferable lower limit of the pH value is 5.5, the preferable upper limit of the pH value is 8.5, and the more preferable lower limit of the pH value is 6.5.

The antiphospholipid antibody detection reagent of the present invention may contain a water soluble polymer to improve the detection sensitivity and promote the antigen-antibody reaction.
The water soluble polymer is not particularly limited, and examples thereof include pullulan and polyvinyl pyrrolidone.

Yet another aspect of the present invention is a method of detecting an antiphospholipid antibody, the method comprising: mixing a sample and an antiphospholipid antibody detection reagent that contains a buffer solution and an insoluble carrier supporting a phospholipid antigen to cause an antigen-antibody reaction that involves agglutination of the carrier; and detecting a lipid antibody in the sample by optically measuring or visually observing the degree of the agglutination, wherein the insoluble carrier before supporting a phospholipid antigen has a zeta potential of lower than -45 mV in the case that the insoluble carrier is suspended in a 20 mmol/L aqueous sodium phosphate solution with a pH of 7.4 so that the resulting suspension has a solids concentration of 0.1%.

The method of optically measuring the degree of the agglutination is not particularly limited, and may be a conventional method. Examples of such a method include a method of measuring the increase/decrease in the scattered light intensity, light absorbance, or transmission light intensity. The method to be used depends on the particle size of the insoluble carrier to be used, the selected concentration, and the set reaction time. Alternatively, those methods may be used in combination.
The light wavelength for the above measurement is preferably 300 to 900 nm.

In the case of using the method of measuring the change in the light absorbance, the reagent is required, for accurate measurement, to have a sufficient reactivity to show an amount of change in the light absorbance of at least 250 mAbs when mixed with a standard serum having a highest concentration of 8.0 R.U. (R.U. is a unit of measuring the syphilis antibody titer, and a value of 1.0 R.U. or higher indicates positive for syphilis) . If the amount of change in the light absorbance is 250 mAbs at a concentration of 8.0 R.U., the amount of change in the light absorbance at a concentration of 1.0 R.U., which is the boundary value for determining whether the sample tests positive or negative for the antibody in the test with the antiphospholipid antibody detection reagent, should be about 30 mAbs. If the amount of change in the light absorbance results in 30 mAbs or smaller at a concentration of 1.0 R.U., the data reproducibility may be significantly decreased, and thus correct positive/negative determination may not be made.

The device for use in the method of optically measuring the degree of the agglutination is not particularly limited, and examples thereof include optical devices capable of detecting properties such as the scattered light intensity, transmission light intensity, or light absorbance. Any commonly used biochemical autoanalyzer may be used.

The method of visually observing the degree of the agglutination may be, usually, a method of mixing a sample and the antiphospholipid antibody detection reagent of the present invention on a test plate, and then shaking the plate to mix the substances and determine whether agglutination is present.
Alternatively to visual observation, observation of the degree of the agglutination may be performed by a method of recording the agglutination state on video or the like, and then processing the images.

### EFFECT OF THE INVENTION

The present invention can provide an insoluble carrier for an antiphospholipid antibody detection reagent having a high reactivity. The present invention can also provide an antiphospholipid antibody detection reagent and a method of detecting an antiphospholipid antibody.

### BRIEF DESCRIPTION OF DRAWINGS

[Fig. 1]

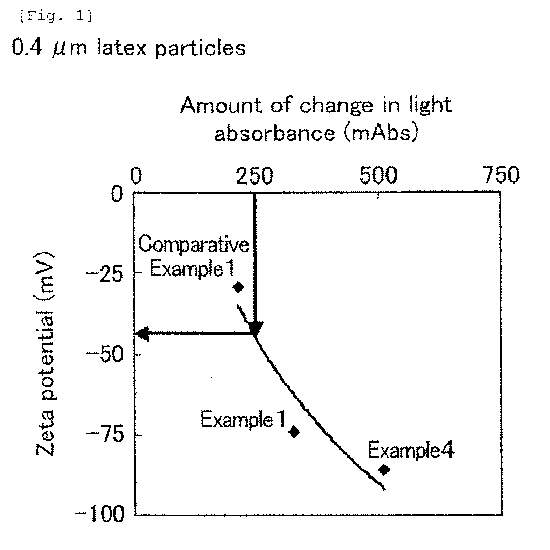

Fig. 1 is a graph showing amounts of change in the light absorbance in Example 1, Example 4, and Comparative Example 1, in the case of using an 8.0 R.U. RPR standard serum.

### MODE(S) FOR CARRYING OUT THE INVENTION

The present invention is described below in more detail with reference to Examples. The present invention is not limited to those Examples.

### (1) Latex Lot A

### (Production of latex particles)

To a glass reaction vessel (volume: 2 L) provided with a stirrer, a reflux condenser, a temperature sensor, a nitrogen inlet tube, and a jacket, 1100 g of distilled water, 180 g of styrene, 0.04 g of sodium styrenesulfonate, and an aqueous solution produced by dissolving 0.8 g of potassium persulfate in 26 g of distilled water were charged. Thereafter, the atmosphere in the vessel was replaced by nitrogen gas, and then polymerization was allowed to proceed for 48 hours at 70°C while the solution was stirred.
After completion of the polymerization, the solution was filtered by filter paper to separate latex particles. The particle sizes, the amount of sulfonic acid group per unit surface area, and the zeta potential of the produced latex particles were measured by the following methods.

### (Average particle size of latex particles)

The latex particles were photographed by a transmission electron microscope ("JEM-1010", a product of JEOL Ltd.) at 10000 times magnification, and at least 100 particles in the image were analyzed to measure the particle sizes. The average particle size of those particles was 0.4 µm.

(Amount of sulfonic acid group per unit surface area of latex particles)
The latex particles were dialyzed against purified water for 48 hours with a dialysis membrane of a cellophane tube, so that the residual monomers were removed. The particles were extracted to 10 g in dry weight in a 4-necked glass vessel, and were diluted to 150 mL with distilled water. Then, the solution was stirred by a stirrer chip. This resulting solution is referred to as a solution A.
Next, 0.01 N sodium hydroxide (produced by Wako Pure Chemical Industries, Ltd.) aqueous solution was poured into an ATB-310 electric buret, an attachment device of an automatic potentiometric titrator ("AT-310" produced by Kyoto Electronics Manufacturing Co., Ltd.). Also, a conductive electrode was immersed in the solution A, and a nitrogen introducing pipe, a deaeration pipe, and a pH electrode were set up. Thereafter, the 0.01 N sodium hydroxide aqueous solution was dropped (0.05 mL of the solution from the buret for a period in the range of 150 to 500 seconds, the period depending on the amount of sulfonic acid group to be measured), and the equivalence point was measured from the changes in the conductivity measured by an automatic potentiometric titrator ("AT-310" produced by Kyoto Electronics Manufacturing Co., Ltd.), and then the amount of sulfonic acid group was finally calculated. The calculated amount of sulfonic acid group was 0.28 µmol/m²

### (Zeta potential of latex particles)

The latex particles were suspended in a 20 mmol/L aqueous sodium phosphate solution with a (pH of 7.4 so that the resulting suspension had a solids concentration of 0.1%, and the suspension was taken as a zeta potential measurement sample.
Next, in a zeta potential measuring device ("Zetasizer Nano ZEN3600", a product of Malvern Instruments Ltd.), 750 µL of the measurement sample was injected into a capillary cell for zeta potential measurement, and the zeta potential of the sample was measured at a measurement temperature of 37°C. The measured zeta potential of the sample was -74 mV.

### (2) Latex Lot B

The latex particles were produced by the same procedure as that for Lot A, except the blending amount of sodium styrenesulfonate was 0.08 g. The latex particles were evaluated by the same methods as those for Lot A. The average particle size of the latex particles was 0.3 µm, the amount of sulfonic acid group was 0.23 µmol/m², and the zeta potential was -77 mV.

### (3) Latex Lot C

The latex particles were produced by the same procedure as that for Lot A, except the blending amount of distilled water was 1020 g, the blending amount of sodium styrenesulfonate was 0.25 g, an aqueous solution produced by dissolving 0.4 g of potassium persulfate in 13 g of distilled water was used instead of an aqueous solution produced by dissolving 0.8 g of potassium persulfate in 26 g of distilled water, and 80 g of 0.1 mol/L dipotassium hydrogen phosphate was added. The latex particles were evaluated by the same methods as those for Lot A. The average particle size of the latex particles was 0.3 µm, the amount of sulfonic acid group was 0.16 µmol/m², and the zeta potential was -88 mV.

### (4) Latex Lot D

The latex particles were produced by the same procedure as that for Lot A, except the blending amount of distilled water was 1020 g, the blending amount of sodium styrenesulfonate was 0.20 g, an aqueous solution produced by dissolving 0.6 g of potassium persulfate in 16 g of distilled water was used instead of an aqueous solution produced by dissolving 0.8 g of potassium persulfate in 26 g of distilled water, and 80 g of 0.1 mol/L dipotassium hydrogen phosphate was added. The latex particles were evaluated by the same methods as those for Lot A. The average particle size of the latex particles was 0.4 µm, the amount of sulfonic acid group was 0.18 µmol/m², and the zeta potential was -86 mV.

### (5) Latex Lot E

The latex particles were produced by the same procedure as that for Lot A, except the blending amount of sodium styrenesulfonate was 0.01 g, and an aqueous solution produced by dissolving 0.1 g of potassium persulfate in 4 g of distilled water was used instead of an aqueous solution produced by dissolving 0.8 g of potassium persulfate in 26 g of distilled water. The latex particles were evaluated by the same methods as those for Lot A. The average particle size of the latex particles was 0.4 µm, the amount of sulfonic acid group was 0. 06 µmol/m², and the zeta potential was -29 mV.

### (Example 1)

### (1) Preparation of buffer solution (first reagent)

A buffer solution (first reagent) was prepared by adding 0.9 g of sodium chloride and 0.1 g of sodium azide into 100 mL of a 25 mmol/L phosphate buffer solution (pH 6.5) that contains 1.2(W/V)% pullulan (produced by Hayashibara Co., Ltd.) and 1.0(W/V)% bovine serum albumin (BSA).

### (2) Preparation of phospholipid-antigen sensitized latex reagent (second reagent)

A phospholipid antigen solution was prepared by mixing 2 mL of an ethanol solution of cardiolipin (5 mg/mL, a product of Sigma-Aldrich Co.) , 10 mL of an ethanol solution of refined lecithin (10 mg/mL, a product of Nacalai Tesque, Inc.), and 3 mL of an ethanol solution of cholesterol (10 mg/mL, a product of Nacalai Tesque, Inc.).
Thereafter, 250 µL of the resulting phospholipid antigen solution was added to 100 µL of a latex particle (Lot A) suspension, and the mixture was gently stirred at 37°C for two hours. Next, 3 mL of a 100 mmol/L phosphate buffer solution (pH 6.5) containing 5 (W/V) % BSA was added to the mixture, and the mixture was further stirred at 37°C for one hour. The mixture was then centrifuged at 15000 rpm and 4°C for 30 minutes so that the supernatant was removed, and the precipitated latex particles were suspended again in 2 mL of a 100 mmol/L phosphate buffer solution (pH 6.5) containing 1(W/V)% BSA. The above process was repeated twice. Then, the latex particles were washed, and suspended in 10 mL of a 100 mmol/L phosphate buffer solution (pH 6.5) that contains 10 mmol/L EDTA·4Na and 500 mmol/L choline chloride. Thereby, a phospholipid-antigen sensitized latex reagent (second reagent) was produced.

### (Example 2)

A buffer solution (first reagent) and a phospholipid-antigen sensitized latex reagent (second reagent) were prepared by the same procedures as those for Example 1, except the latex particles were changed to Lot B.

### (Example 3)

A buffer solution (first reagent) and a phospholipid-antigen sensitized latex reagent (second reagent) were prepared by the same procedures as those for Example 1, except the latex particles were changed to Lot C.

### (Example 4)

A buffer solution (first reagent) and a phospholipid-antigen sensitized latex reagent (second reagent) were prepared by the same procedures as those for Example 1, except the latex particles were changed to Lot D.

### (Comparative Example 1)

A buffer solution (first reagent) and a phospholipid-antigen sensitized latex reagent (second reagent) were prepared by the same procedures as those for Example 1, except the latex particles were changed to Lot E.

### <Evaluation>

The buffer solution (first reagent) and phospholipid-antigen sensitized latex reagent (second reagent) produced in each of Examples and Comparative Example were evaluated by the following method.
An amount of 180 µL of the buffer solution (first reagent) was mixed into 20 µL of a commercially available 8.0 R.U. RPR standard serum (a product of Sekisui Medical Co., Ltd.), and the mixture was allowed to stand at 37°C for five minutes. Thereafter, 60 µL of the phospholipid-antigen sensitized latex reagent (second reagent) was added to the mixture, and the mixture was stirred. The amount of change in the light absorbance was determined by measuring the light absorbance of the mixture one minute after the addition and five minutes after the addition, by a Hitachi biochemistry automatic analyzer 7170 at a measurement wavelength of 700 nm. Table 1 and Fig. 1 show the results.

Fig. 1 shows that a zeta potential of -45 mV or higher did not result in the required amount of change in the light absorbance, and that a lower zeta potential leads to a larger amount of change in the light absorbance.

**[Table 1]**

| | Average particle size (µm) | Zeta potential (mV) | Amount of change in light absorbance (mAbs) |
|---|---|---|---|
| Example 1 | 0.4 | -74 | 323 |
| Example 2 | 0.3 | -77 | 427 |
| Examples 3 | 0.3 | -88 | 496 |
| Examples 4 | 0.4 | -86 | 509 |
| Comparative Example 1 | 0.4 | -29 | 214 |

### INDUSTRIAL APPLICABILITY

The present invention can provide an insoluble carrier for an antiphospholipid antibody detection reagent having a high reactivity. The present invention can also provide an antiphospholipid antibody detection reagent, and a method of detecting an antiphospholipid antibody.

## Claims

1. An insoluble carrier for an antiphospholipid antibody detection reagent,
which has a zeta potential of lower than -45 mV in the case that the insoluble carrier is suspended in a 20 mmol/L aqueous sodium phosphate solution with a pH of 7.4 so that the resulting suspension has a solids concentration of 0.1%.

2. The insoluble carrier according to claim 1, wherein the insoluble carrier has an average particle size of 0.2 µm to 0.5 µm.

3. An antiphospholipid antibody detection reagent for antiphospholipid antibody detection,
which comprises:
an insoluble carrier supporting a phospholipid antigen; and
a buffer solution,
wherein the insoluble carrier before supporting a phospholipid antigen has a zeta potential of lower than -45 mV in the case that the insoluble carrier is suspended in a 20 mmol/L aqueous sodium phosphate solution with a (pH of 7.4 so that the resulting suspension has a solids concentration of 0.1%.

4. The antiphospholipid antibody detection reagent according to claim 3,
wherein the insoluble carrier has an average particle size of 0.2 µm to 0.5 µm.

5. A method of detecting an antiphospholipid antibody, the method comprising:
mixing a sample and an antiphospholipid antibody detection reagent that contains a buffer solution and an insoluble carrier having a phospholipid antigen to cause an antigen-antibody reaction that involves agglutination of the carrier; and
detecting a lipid antibody in the sample by optically measuring or visually observing the degree of the agglutination,
wherein the insoluble carrier before supporting a phospholipid, antigen has a zeta potential of lower than -45 mV in the case that the insoluble carrier is suspended in a 20 mmol/L aqueous sodium phosphate solution with a pH of 7.4 so that the resulting suspension has a solids concentration of 0.1%.

6. The method of detecting an antiphospholipid antibody according to claim 5,
wherein the insoluble carrier has an average particle size of 0.2 µm to 0.5 µm.

## Amended claims

### Amended claims under Art. 19.1 PCT

1. An insoluble carrier for an antiphospholipid antibody detection reagent,
which has a zeta potential of lower than -45 mV in the case that the insoluble carrier is suspended in a 20 mmol/L aqueous sodium phosphate solution with a pH of 7.4 so that the resulting suspension has a solids concentration of 0.1%.

2. The insoluble carrier according to claim 1, wherein the insoluble carrier has an average particle size of 0.2 µm to 0.5 µm.

3. An antiphospholipid antibody detection reagent for antiphospholipid antibody detection,
which comprises:
an insoluble carrier supporting a phospholipid antigen; and
a buffer solution,
wherein the insoluble carrier before supporting a phospholipid antigen has a zeta potential of lower than -45 mV in the case that the insoluble carrier is suspended in a 20 mmol/L aqueous sodium phosphate solution with a pH of 7.4 so that the resulting suspension has a solids concentration of 0.1%.

4. The antiphospholipid antibody detection reagent according to claim 3,
wherein the insoluble carrier has an average particle size of 0.2 µm to 0.5 µm.

5. A method of detecting an antiphospholipid antibody, the method comprising:
mixing a sample and an antiphospholipid antibody detection reagent that contains a buffer solution and an insoluble carrier having a phospholipid antigen to cause an antigen-antibody reaction that involves agglutination of the carrier; and
detecting a lipid antibody in the sample by optically measuring or visually observing the degree of the agglutination,
wherein the insoluble carrier before supporting a phospholipid antigen has a zeta potential of lower than -45 mV in the case that the insoluble carrier is suspended in a 20 mmol/L aqueous sodium phosphate solution with a pH of 7.4 so that the resulting suspension has a solids concentration of 0.1%.

6. The method of detecting an antiphospholipid antibody according to claim 5,
wherein the insoluble carrier has an average particle size of 0.2 µm to 0.5 µm.

7. The insoluble carrier according to claim 1, wherein the insoluble carrier does not contain an emulsifier.

8. The insoluble carrier according to claim 1, wherein the insoluble carrier has a zeta potential of -74 mV or lower in the case that the insoluble carrier is suspended in a 20 mmol/L aqueous sodium phosphate solution with a pH of 7.4 so that the resulting suspension has a solids concentration of 0.1%.
